# FASCICULE DE BREVET EUROPEEN

(11) **EP 3 126 508 B1**
(45) Date de publication et mention de la délivrance du brevet: **08.07.2026**
(21) Numéro de dépôt: 15719804.5
(22) Date de dépôt: 03.04.2015
(51) Int. Cl.: C12P 7/64, A23K 10/40, C12N 1/12

(54) **PROCÉDÉ DE CULTURE DES MICROALGUES DU GENRE AURANTIOCHYTRIUM DANS UN MILIEU DE CULTURE SANS CHLORURE ET SANS SODIUM POUR LA PRODUCTION DE DHA**
VERFAHREN ZUR KULTUR MIKROALGE DER GATTUNG AURANTIOCHYTRIUM IN EINEM KULTURMEDIUM OHNE NATRIUM UND OHNE CHLORID FÜR DIE HERSTELLUNG VON DHA
PROCESS FOR CULTURING MICROALGAE OF THE GENUS AURANTIOCHYTRIUM IN A CULTURE MEDIUM WITHOUT SODIUM AND WITHOUT CHLORIDE FOR THE PRODUCTION OF DHA

(30) Priorité: 03.04.2014 FR 1452960
(43) Date de publication de la demande: 08.02.2017
(73) Titulaire: Fermentalg, 33500 Libourne (FR)
(72) Inventeur: CALLEJA, Pierre, F-33600 Libourne (FR); PAGLIARDINI, Julien, F-33300 Bordeaux (FR); CAGNAC, Olivier, F-33500 Libourne (FR); GODART, François, F-33870 Vayres (FR)
(74) Mandataire: Icosa
(86) Numéro de dépôt international: PCT/FR2015/050881
(87) Numéro de publication internationale: WO 2015/150716

(56) Documents cités:
- WO-A1-2005/035775
- US-A1- 2007 054 384
- US-B1- 6 410 281
- KAI-CHUANG CHAUNG ET AL: "Effect of culture conditions on growth, lipid content, and fatty acid composition of Aurantiochytrium mangrovei strain BL10", AMB EXPRESS, vol. 2, no. 1, 1 January 2012 (2012-01-01), pages 42, XP055078009, ISSN: 2191-0855, DOI: 10.1186/2191-0855-2-42
- DATABASE EMBL [online] 22 September 2009 (2009-09-22), "Aurantiochytrium sp. BL10 18S ribosomal RNA gene, partial sequence.", retrieved from EBI accession no. EM_STD:FJ821477 Database accession no. FJ821477
- HUEY-LANG YANG ET AL: "Isolation and Characterization of Taiwanese Heterotrophic Microalgae: Screening of Strains for Docosahexaenoic Acid (DHA) Production", MARINE BIOTECHNOLOGY, SPRINGER-VERLAG, NE, vol. 12, no. 2, 16 July 2009 (2009-07-16), pages 173 - 185, XP019784661, ISSN: 1436-2236
- RINKA YOKOYAMA ET AL: "Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and erection of Aurantiochytrium and Oblongichytrium gen. nov", MYCOSCIENCE, SPRINGER-VERLAG, TO, vol. 48, no. 4, 6 August 2007 (2007-08-06), pages 199 - 211, XP019521654, ISSN: 1618-2545, DOI: 10.1007/S10267-006-0362-0
- SULEEPORN KITCHA ET AL: "Screening of Oleaginous Yeasts and Optimization for Lipid Production Using Crude Glycerol as a Carbon Source", ENERGY PROCEDIA, vol. 9, 2011, pages 274 - 282, XP028105725, ISSN: 1876-6102, [retrieved on 20111107], DOI: 10.1016/J.EGYPRO.2011.09.029
- AHMAD ISKANDAR BIN HAJI MOHD TAHA ET AL: "Growth optimization of thraustochytrid strain 12B for the commercial production of docosahexaenoic acid", FOOD SCIENCE AND BIOTECHNOLOGY, vol. 22, no. S1, 1 February 2013 (2013-02-01), pages 53 - 58, XP055143597, ISSN: 1226-7708, DOI: 10.1007/s10068-013-0048-2
- LANA SHABALA ET AL: "Thraustochytrids Can Be Grown in Low-Salt Media Without Affecting PUFA Production", MARINE BIOTECHNOLOGY, vol. 15, no. 4, 9 April 2013 (2013-04-09), pages 437 - 444, XP055143634, ISSN: 1436-2228, DOI: 10.1007/s10126-013-9499-y

## Description

L'invention se rapporte à un procédé de culture de cellules de Thraustochytrides du genre *Aurantiochytrium mangrovei.* Le procédé permet d'obtenir un haut rendement en biomasse et un enrichissement des protistes ainsi cultivés en lipides et plus particulièrement en acide docosahexaénoïque (DHA). L'invention concerne la mise au point d'un procédé de culture permettant la production à haute densité cellulaire de Thraustochytrides riches en DHA sur milieu chimiquement défini à teneur réduite en ions de sodium (Na⁺) et de chlorure (Cl⁻).

L'invention concerne seulement les Thraustochytrides appartenant au genre *Aurantiochytrium.* Ce genre est délimité par une caractérisation génétique, métabolique et physiologique.

Le procédé de culture, par lequel les cellules sont cultivées sans ajout significatif d'ions de sodium (Na⁺), ni de chlorure (Cl⁻), permet la production à haute densité cellulaire - environ 125 à 140 g/L de matière sèche - d'*Aurantiochytrium mangrovei.* Cette biomasse est riche en DHA, avec un taux de 15 à 20 g/L de culture, de préférence 20 à 25 g/L de culture, ou même 25 à 30 g/L de culture.

De très faibles quantités de chlorure de sodium sont présentes dans le milieu de culture nécessaire pour ce procédé, spécifiquement, de très faibles quantités d'ions de chlorure (Cl⁻) et de sodium (Na⁺). Ainsi, dans le milieu de culture, il y a moins de 1 g/L, de préférence moins de 0,5 g/L, plus préférentiellement moins de 0,2 g/L d'ions de chlorure, et moins de 100 mg/L, de préférence moins de 50 mg/L et plus préférentiellement moins de 6 mg/L d'ions de sodium (Na⁺). Cela permet de s'affranchir des surcoûts d'investissement nécessaires pour les équipements en contact avec le milieu et de réduire substantiellement les coûts de traitement des effluents, de s'affranchir des inconvénients associés à la présence de sel de sodium ou de chlorure dans la biomasse et de réduire le coût du milieu en diminuant les intrants (des produits ajoutés à la culture pour améliorer le rendement).

### Préambule

Les protistes font l'objet actuellement de nombreux projets industriels car certaines espèces sont capables d'accumuler ou de secréter des quantités importantes de lipides, notamment d'acides gras polyinsaturés.

Parmi les acides gras polyinsaturés, certains hautement insaturés (AGHI) de la série des oméga-3 (PUFA-ω3), en particulier l'acide éicosapentaénoïque (EPA ou C20:5 ω3) et l'acide docosahexaénoïque (DHA ou C22:6 ω3), et de la série des oméga-6 (PUFA-ω6), en particulier, l'acide arachidonique (ARA ou AA ou encore acide eicosatétraénoïque C20:4 ω6), ont une importance nutritionnelle reconnue et présentent de fortes potentialités en terme d'applications thérapeutiques.

Considéré comme nutriment essentiel, le DHA est nécessaire au développement normal et fonctionnel des cellules, et joue un rôle crucial dans divers processus et fonctions biochimiques. Il est indispensable au développement du système nerveux central et à la fonction rétinienne, par incorporation dans les membranes cellulaires, et joue un rôle capital dans l'acquisition et le maintien satisfaisant des mécanismes de la vision et de la mémoire.

Les Thraustochytrides, en particulier *Aurantiochytrium,* sont connus pour produire du DHA lorsqu'ils sont cultivés en hétérotrophie [W.K. Hong et al. (2011); Production of lipids containing high levels of docosahexaenoic acid by a newly isolated microalga, Aurantiochytrium sp. KRS101. Appl. Biochem. Biotechnol.: 164(8):1468-80]. *Aurantiochytrium* est aussi connue pour produire des caroténoïdes, tels que l'astaxanthine, la zéaxanthine, la canthaxanthine, l'échinénone, le bêta-carotène et la phoenicoxanthine [Yokoyama, R, Honda, D. (2007) Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and erection of Aurantiochytrium and Oblongichytrium gen. nov.; Mycoscience, Vol. 48, pp. 199-211].

Pour mettre en œuvre la production des acides gras par des protistes à l'échelle industrielle, plusieurs facteurs doivent être pris en compte pour rendre la production rentable. Parmi ces facteurs, on peut citer :
- les coûts des matières premières et des équipements (leur achat ou location ainsi que leur maintenance), ainsi que de la main d'œuvre ;
- les exigences techniques de la production: par exemple, le nombre et la difficulté technique des étapes de pré-culture et de culture, le suivi en ligne des cultures, et les étapes de traitement de la biomasse issue de la culture pour valoriser le produit ;
- le traitement des effluents issus de la culture.

Les milieux de culture utilisés actuellement pour cultiver des protistes de la famille des Thraustochytrides en hétérotrophie ou en mixotrophie, contiennent des quantités significatives de sel, en particulier de chlorure de sodium. A titre d'exemple, on peut citer le milieu de culture ATCC medium N° 790 (11 g/L Na⁺ et 19 g/L Cl⁻).

L'utilisation de chlorure de sodium (NaCl) pour la culture des protistes marins de la famille des Thraustochytrides dans les procédés de production d'huile et/ou autres molécules d'intérêt entraîne des surcoûts importants en termes d'investissement, de traitement des effluents et limite la valorisation des coproduits.

En effet, les ions chlorure provoquent une dégradation de l'acier inoxydable, une matière utilisée pour la fabrication des fermenteurs, des outils de préparation et de stérilisation du milieu de culture et autres équipements pour la culture des microalgues et le traitement de la biomasse résultante. Une des conséquences de ce phénomène est la dégradation précoce des outils de production et du traitement de la biomasse (« Down-Stream Processing » ou « DSP » en anglais).

Pour éviter ce problème de dégradation des équipements, on peut utiliser des équipements d'alliages particuliers plus résistants aux ions chlorure. Ces matières plus résistantes au sel sont plus coûteuses. Dans ce cas, les coûts d'investissement pour la production sont substantiellement accrus.

Par ailleurs, l'utilisation de chlorure de sodium (ou d'autres sels de sodium, par exemple de type sulfate de sodium, carbonate de sodium) entraîne des surcoûts importants en terme de traitement des effluents, en particulier la désalinisation de l'eau.

Enfin, la présence de sels de sodium dans les coproduits de type tourteaux constitués par la biomasse restante après extraction de l'huile gêne leur valorisation, notamment pour la nutrition animale, pisciculture, ou en tant qu'ingrédient pour la cosmétique ou dans l'industrie pharmaceutique.

US 5 518 918 décrit le remplacement du chlorure de sodium par d'autres types de sels de sodium (sulfate de sodium, carbonate de sodium...). Même si cela permet de s'affranchir de l'usure précoce des équipements en acier inoxydable, l'ajout des sels de sodium au milieu ne permet pas de s'affranchir du surcoût lié au traitement des effluents, ni des problèmes de valorisation des coproduits indiqués plus haut.

En outre, le remplacement du NaCl par un autre sel de sodium entraîne un surcoût lié à l'achat du sel de substitution.

Dans l'article intitulé « Optimization of docosahexaenoic acid production by Schizochytrium limacinum SR21 » de Yokochi et al. [(1998) Appl. Microbiol. Vol. 49, pp. 72-76], la tolérance de la souche *Schizochytrium limacinum* SR21 pour des conditions de sels a été étudiée. La souche avait une tolérance importante pour de fortes concentrations en sel, la concentration étant comprise entre 50% et 200% de celle de l'eau de mer. La croissance de la souche dans la culture sans sel était deux fois moins importante que dans la culture contenant 50% d'eau de mer. Nous notons que le milieu de culture de base utilisé dans cette étude comprenait également 3% de glucose et 1% d'extrait de levure. Un milieu de culture allégué « sans sel » ou à « faible concentration » en sels est décrit dans le brevet US 8,900,831. Toutefois, comme pour Yokoshi & al., l'ajout d'extrait de levure est nécessaire à la croissance des microalgues. Or, un tel milieu supplémenté en extrait de levure comprend plus de 30 mg de sels de sodium et de chlore.

Au surplus, l'ajout de l'extrait de levure représente un coût supplémentaire pour le milieu, et également un désavantage par rapport à la qualité de la biomasse finale pour son utilisation en tant que produit alimentaire ou pharmaceutique. Les extraits de levures ne sont pas des produits standardisés et donc les lots d'extraits de levures ne sont pas homogènes. Ceci a ainsi un impact sur l'homogénéité des produits finaux issus de la biomasse d'un milieu de culture contenant des extraits de levures.

Shabala et al. [« Osmotic adjustment and requirement for sodium in marine protist thraustochytrid » (2009) Environmental Microbiology Vol. 11(7), pp. 1835-1843] a démontré que *Thraustochytrium* peut croître dans le milieu de culture à teneur réduite en sodium (1 mM), à condition que le milieu soit supplémenté avec un composé tel que le mannitol ou le saccharose, qui permet l'ajustement osmotique de ce milieu de culture sans sodium. Cependant, la présence de ces derniers composés entraîne des surcoûts associés au coût de ces matières. D'ailleurs, malgré l'ajout des composés qui ajustent l'osmolarité du milieu de culture, les rendements de biomasse (200 000 cellules par millilitre) obtenus sans sel et avec du mannitol restent insuffisants pour une production industrielle du DHA.

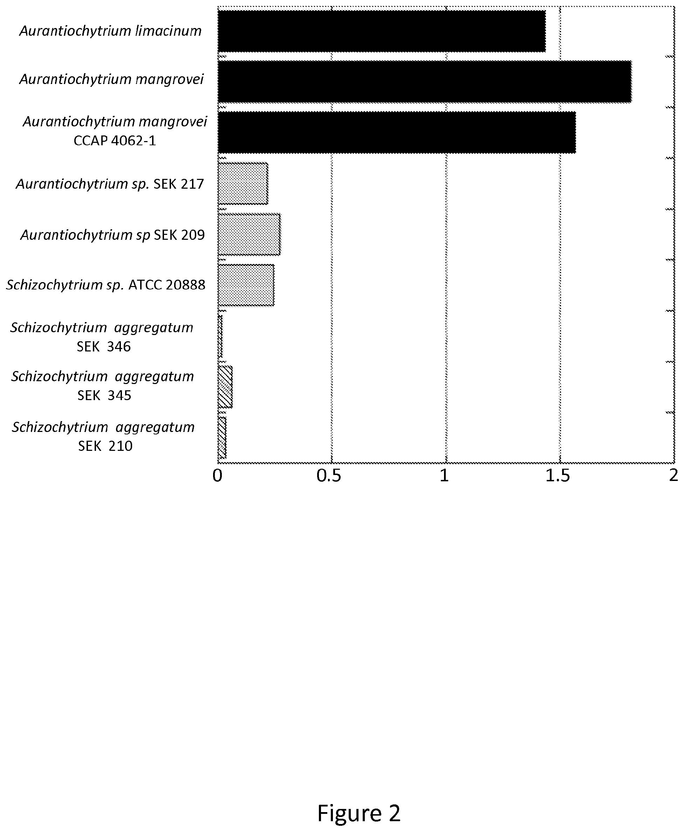

Dans un article récent de Shabala et al. [« Thraustochytrids can be grown in low-salt media without affecting PUFA production », Marine Biotechnology (2013) 15: 437-444], un mutant a été obtenu par mutagénèse aléatoire aux UVs de *Schizochytrium limacinum* SR21 - nommé OUC88 - et testé pour déterminer les facteurs environnementaux qui induisent un changement dans la composition des acides gras. La figure 2 de l'article montre que, dès que la concentration de sel passe en-dessous de 0,9%, la biomasse et la quantité de lipides diminuent fortement (moins de 20 g/L en biomasse et moins de 10 g/L en lipides).

Chen et al. (AMB Express (2012) 2 : 42) décrit l'impact de la salinité du milieu de culture sur la teneur en acides gras de la biomasse produite à partir *d'Aurantiochytrium mangrovei.*

Il est souhaitable de pouvoir cultiver les Thraustochytrides dans des conditions optimales pour augmenter le rendement de(s) l'acide(s) gras à produire, tout en évitant les problèmes liés à l'usure des équipements en acier, en réduisant les coûts de la production pour la fermentation, ainsi que pour le traitement de la biomasse résultante. En particulier, il est souhaitable de fournir des méthodes de culture *d'Aurantiochytrium* qui permettent de réduire, voire substantiellement d'éliminer, les ions de sodium et de chlorure des milieux de culture, et ceci sans l'ajout d'autre(s) constituant(s) de culture, qui peuvent entraîner des surcoûts liés au traitement des effluents, ainsi que des surcoûts liés à des étapes supplémentaires de DSP et des problèmes de valorisation des produits finaux.

Dans le contexte de la présente invention il est souhaitable d'obtenir un rendement en biomasse et en lipides suffisant pour une production industrielle du DHA. Il est donc souhaitable d'obtenir des rendements de, par exemple, supérieurs à 100 g/L de matière sèche, de préférence supérieurs à 130 g/L de matière sèche, plus préférentiellement encore supérieurs à 150 g/L de matière sèche. Il est donc souhaitable d'obtenir, par exemple, plus de 40%, voire 50% d'acides gras par rapport au poids total de la matière sèche. Il est aussi souhaitable d'obtenir, par exemple, plus de 30%, voire 40% de DHA dans les acides gras par rapport au poids total de la matière sèche.

Ainsi, c'est au terme de nombreuses expérimentations de criblage de souches que le demandeur est parvenu à identifier des souches de protiste du genre *Aurantiochytrium,* capables de croître dans un milieu de culture chimiquement défini sans ajout de sodium, ni de chlorure, ni d'une source organique telle que de l'extrait de levure, ni d'agents osmotiques tels que du mannitol, ou du saccharose, comme définis dans les articles de Shabala et *al.* (2013) (ou d'autres agents osmotiques tels que le sorbitol, le polyéthylène glycol PEG).

Ces souches, cultivées dans les conditions de la présente invention, permettent d'obtenir une production à haut rendement en biomasse (supérieur à 110 g/L, de préférence 120 g/L) et en acides gras polyinsaturés (supérieur à 15 g/L, de préférence 20 g/L), notamment en DHA.

Une souche (FCC 1324), concernée par l'invention, représentative des souches d*'Aurantiochytrium* ainsi isolées et sélectionnées, a été déposée auprès de la CCAP (Culture Collection of Algae and Protozoa, Scottish Association for Marine Science, Dunstaffnage Marine Laboratory, Oban, Argyll PA371QA, Ecosse, Royaume-Uni) le 21 juin 2013, selon les dispositions du Traité de Budapest, sous le numéro d'accession CCAP 4062/1.

Ainsi, l'invention est un procédé de production de DHA comprenant l'étape suivante :
la culture, en conditions hétérotrophes ou mixotrophes, d'une ou plusieurs souches du genre *Aurantiochytrium* dans un milieu de culture ayant moins de 3,5 g/L d'ions de sodium,
caractérisé en ce que les souches du genre *Aurantiochytrium* ont une identité génétique d'au moins 92% à la séquence SEQ NO. 1 et
en ce que le milieu de culture est un milieu chimiquement défini ayant moins de 3,5 g/L d'ions de sodium et moins de 1 g/L d'ions de chlorure et ayant de 200 mM à 500 mM de substrat carboné organique.

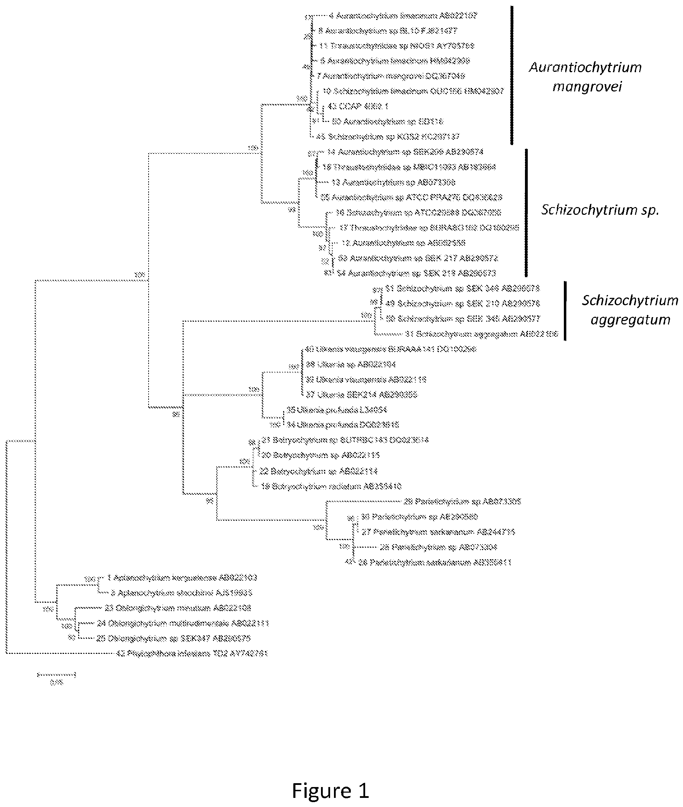

**Figure 1****: Analyse phylogénétique montrant les relations entre les séquences d'ADN codant la petite sous-unité de l'ARN ribosomique.** Les séquences ont été alignées avec ClustalW de Méga 5.1. L'analyse a été conduite en utilisant la méthode Maximum Likelihood. Les souches de Thraustochytrides utilisées dans cette étude appartiennent aux genres: *Aurantiochytrium mangrovei, Schizochytrium sp,* et *Schizochytrium aggregatum, Ulkenia visurgensis, Ulkenia sp, Ulkenia profunda, Botryochytrium sp., Botryochytriumradiatum, Parieticytrium sp., Parieticytrium sarkarianum, Aplanochtytrium kerguelense,, Aplanochtytrium stocchinoi, Oblongichytrium multirudimentale, Oblongichytrium sp. et Phytophthora infestans.* Les valeurs de bootstrap sont considérées comme significatives si elles sont supérieures à 75%.
**Figure 2****: Tests de croissance en Erlen avec le milieu FCC-M à teneur réduite en Na⁺ et Cl⁻.** Comparaison de la croissance des souches concernées par l'invention (en noir) et non concernées par l'invention (en gris et traits) dans un milieu de culture selon un mode de réalisation de l'invention. La longueur de la colonne pour chaque souche représente la densité optique (Exemple 1).

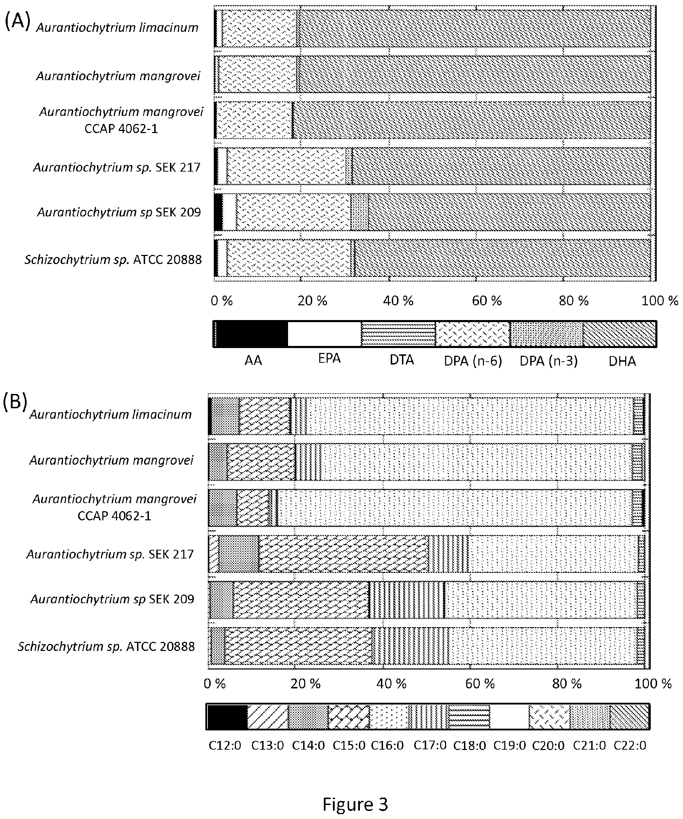

**Figure 3****: Profils en acides gras des différentes souches de Thrautochytrides cultivées en Erlen (Exemple 2).**
   **(A) et (B)** : Une comparaison des profils d'acides gras entre le genre selon l'invention, exemplifié par *Aurantiochytrium mangrovei,* dont les *Aurantiochytrium limacinum* font partie (trois premiers lignes des panneaux), et le genre des *Schizochytrium sp*., qui regroupe les souches ATCC 20888, ainsi que les souches *Aurantiochytrium sp* SEK 217 et SEK 209 (trois dernières lignes des panneaux). Les conditions de culture sont décrites dans l'**Exemple 2.**

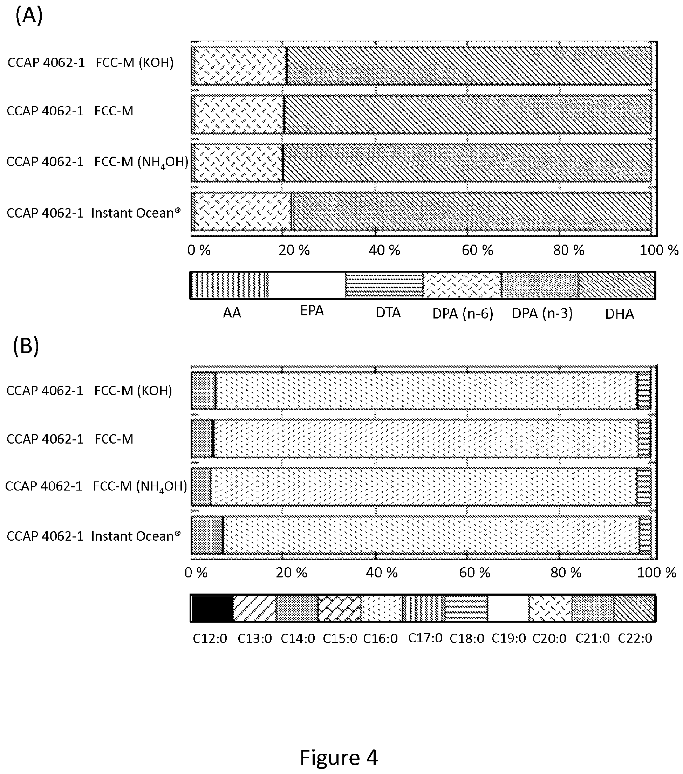

**Figure 4****: Profils en acides gras des différentes souches de Thrautochytrides cultivées en Bioréacteur (Exemples 3 et 4).**
   **(A) et (B)** : Profils lipidiques des souches selon l'invention. (A) Acides gras polyinsaturés (PUFAs) exprimés en pourcentages par rapport aux PUFAs totaux. (B) Acides saturés exprimés en pourcentages par rapport aux acides gras saturés totaux. Les cultures dont le pH a été régulé par de la KOH ou de NH₄OH ont été identifiées respectivement par le sigle (KOH) ou (NH₄OH). Les conditions de culture sont décrites dans les **Exemples 3 et 4.**

### Description détaillée

Par « souche », on entend non seulement les souches naturelles du genre *Aurantiochytrium* défini selon l'invention, mais également les mutants desdites souches naturelles.

Par « chimiquement défini » on entend tout produit ou mélange de produits dont la composition chimique est connue et dont la teneur de chaque élément qui constitue le produit ou le mélange est également connue.

Par « milieu de culture chimiquement défini », on entend milieu de culture dans lequel la teneur de chaque élément est connue, c'est-à-dire en l'absence d'extraits de levures ou d'autres sources complexes de protéines ou autres matières organiques telles que de la peptone ou autre agent de croissance complexe dont la composition est variable tant dans la nature qu'en l'absence de concentration fixe de chacun de ces composants.

Par « agent régulateur osmotique » on entend un agent présent dans un milieu de culture qui permet de maintenir la pression osmotique dans le milieu.

Par « identité génétique », on entend une identité entre deux séquences d'ADN, telle qu'effectuée par un logiciel de type BLAST.

La présente invention a donc pour objet un procédé de culture de certains protistes du type *Aurantiochytrium mangrovei* et *Aurantiochytrium limacinum* en hétérotrophie ou en mixotrophie dans un milieu organique substantiellement libre de sodium (Na⁺) et de chlorure (Cl⁻). Ce procédé de culture permet d'obtenir de hauts rendements en biomasse, en lipides, et spécifiquement en DHA.

Les souches concernées par l'invention ont la capacité de croître à haute densité, dans les milieux de culture chimiquement définis, sans l'ajout de quantités significatives d'ions de sodium ni de chlorure, et sans l'ajout d'agents régulateurs osmotiques, tels que le mannitol, le sorbitol, ou le polyéthylène glycol. Elles sont, selon des classements phylogénétiques récents, des souches de Thraustochytrides de type *Aurantiochytrium mangrovei* et *Aurantiochytrium limacinum,* connues pour être productrices de DHA [Yokoyama R, et al. (2007). Taxonomic rearrangement of the genus Ulkenia sensu lato based on morphology, chemotaxonomical characteristics, and 18S rRNA gene phylogeny (Thraustochytriaceae, Labyrinthulomycetes): emendation for Ulkenia and erection of Botryochytrium, Parietichytrium. Mycoscience. 48(6) p. 329-341; Yokoyama, R., Honda, D. (2007) Taxonomic rearrangement of the genus Schizochytrium sensu lato based on morphology, chemotaxonomical characteristics and 18S rRNA gene phylogeny (Thraustochytriaceae, Labyrinthulomycetes, stramenopiles): emendation for Schizochytrium and erection of Aurantiochytrium and Oblongichytrium gen. Nov. Mycoscience 48, 199-211; Tsui CK, et al. (2009) Labyrinthulomycetes phylogeny and its implications for the evolutionary loss of chloroplasts and gain of ectoplasmic gliding. Mol Phylogenet Evol. 50(1):p. 129-40].

On rappelle que de façon classique, des cultures en mode hétérotrophe de ces genres de microalgues s'effectuaient avec un milieu de culture à base d'eau de mer, comme celui utilisé par l'American Type Culture Collection, le milieu ATCC 790 By+ (extrait de levure 1,0 g, peptone 1,0 g D(+), glucose 5,0 g et eau de mer 1 litre).

Les souches sont caractérisées génétiquement, ainsi que par leur profil lipidique.

Les souches concernées par l'invention sont caractérisées par l'identité génétique de quatre de leurs gènes, 18s, actine, tubuline et EF1-alpha, à des gènes d'une souche représentative des souches d'invention, la souche FCC 1324. La souche FCC 1324 est représentative des nouvelles souches *d'Aurantiochytrium* ainsi isolées et sélectionnées, et a été déposée auprès de la CCAP (Culture Collection of Algae and Protozoa, Scottish Association for Marine Science, Dunstaffnage Marine Laboratory, Oban, Argyll PA371QA, Ecosse, Royaume-Uni) le 21 juin 2013, selon les dispositions du Traité de Budapest, sous le numéro d'accession CCAP 4062/1.

Le **Tableau 1(a)** est un comparatif des séquences des quatre gènes entre le genre *Aurantiochytrium mangrovei* et le genre le plus proche génétiquement *Schizochytrium sp.,* ainsi que d'autres souches proches génétiquement. Toutes les souches présentant des séquences ayant entre 91% et 100% d'identité avec les gènes de la souche CCAP 4062-1, selon les gènes comparés, peuvent être considérées comme étant du genre *Aurantiochytrium.* Une identité génétique d'au moins 92% au gène 18s CCAP 4062-1 considéré caractérise les souches selon l'invention.

**Tableau 1(a)**

| **18s** | | |
|---|---|---|
| **Souches de *Schizochytrium sp.*** | **Longueur des séquences comparées (%)** | **% d'identité avec CCAP 4062-1** |
| Aurantiochytrium_sp_SEK209_AB290574 | 99% | 91% |
| Thraustochytriidae_sp_MBIC11093_AB183664 | 100% | 91% |
| Aurantiochytrium_sp_AB073308 | 94% | 91% |
| Aurantiochytrium_sp_ATCC PRA276 DQ836628 | 93% | 91% |
| Schizochytrium_sp_ATCC20888_DQ367050 | 100% | 91% |
| Thraustochytriidae_sp_BURABG162_DQ100295 | 100% | 91% |
| Aurantiochytrium_sp_AB052555 | 100% | 91% |
| Aurantiochytrium_sp_SEK217 AB290572 | 100% | 91% |
| Aurantiochytrium_sp_SEK 218 AB290573 | 99% | 90% |

| **Souches d'*Aurantiochytrium mangrovei*** | **Longueur des séquences comparées (%)** | **% d'identité avec CCAP 4062-1** |
|---|---|---|
| Aurantiochytrium_limacinum_AB022107 | 77% | 96% |
| Aurantiochytrium_limacinum_HM042909 | 100% | 98% |
| Aurantiochytrium_limacinum_SL1101_JN986842 | 100% | 98% |
| Aurantiochytrium_mangrovei_DQ367049 | 100% | 99% |
| Aurantiochytrium_sp_BL10 FJ821477 | 100% | 98% |
| Aurantiochytrium_sp_LY2012 JX847370 | 100% | 98% |
| Schizochytrium_limacinum OUC166 HM042907 | 100% | 99% |
| Thraustochytriidae_sp_NIOS1_AY705769 | 100% | 99% |
| Thraustochytriidae_sp_LY2012_JX847378 | 83% | 98% |
| Schizochytrium_sp_KGS2_KC297137 | 77% | 98% |
| Aurantiochytrium_sp_LY 2012 PKU Mn5 JX847361 | 100% | 98% |
| Aurantiochytrium_limacinum_JN986842 | 100% | 98% |
| Schizochytrium_sp_SKA10_JQ248009 | 99% | 98% |
| Aurantiochytrium_sp_SD116 | 100% | 99% |

| **Actine** | | |
|---|---|---|
| **Souches** | **Longueur des séquences comparées (%)** | **% d'identité avec CCAP 4062-1** |
| Aurantiochytrium mangrovei DQ356659 | 97% | 99% |
| Schizochytrium sp. ATCC 20888 DQ356660 | 98% | 95% |
| Thraustochytrium aureum DQ356666 | 98% | 90% |
| Japonochytrium marinum DQ356668 | 96% | 89% |
| Thraustochytriidae sp. #32 DQ356663 | 96% | 89% |
| Thraustochytrium aggregatum DQ356662 | 96% | 89% |
| Schizochytrium_aggregatum DQ356661 | 86% | 92% |
| Thraustochytrium striatum DQ356665 | 95% | 88% |
| Thraustochytriidae sp. RT49 DQ356669 | 91% | 89% |

| **EF1** | | |
|---|---|---|
| **Souches** | **Longueur des séquences comparées (%)** | **% d'identité avec CCAP 4062-1** |
| Aurantiochytrium mangrovei | 100% | 99% |
| Schizochytrium sp. ATCC 20888 | 100% | 94% |
| Schizochytrium aggregatum | 100% | 87% |
| Thraustochytrium striatum | 99% | 86% |
| Thraustochytriidae sp. Thel2 | 91% | 87% |
| Thraustochytriidae sp. RT49 | 100% | 84% |

| **Tubuline** | | |
|---|---|---|
| **Souches** | **Longueur des séquences comparées (%)** | **% d'identité avec CCAP 4062-1** |
| Aurantiochytrium mangrovei DQ323157 | 100% | 97% |
| Schizochytrium sp. ATCC 20111 DQ323158* | 100% | 90% |
| Schizochytrium aggregatum DQ323159 | 100% | 87% |
| Japonochytrium marinum DQ323166 | 99% | 87% |
| Thraustochytriidae sp. RT49 DQ323167 | 99% | 87% |
| Thraustochytrium kinnei DQ323165 | 99% | 87% |
| Thraustochytrium striatum DQ323163 | 99% | 86% |
| Thraustochytriidae sp. TheI2 DQ323162 | 99% | 86% |
| Thraustochytriidae sp. #32 DQ323161 | 99% | 85% |
| *Schizochytrium sp. ATCC 20111 DQ323158 = ATCC 20888 | | |

Les souches ayant une identité génétique d'au moins 92% pour les gènes 18s (SEQ NO. 1) sont concernées par l'invention et sont donc susceptibles de croître dans un milieu réduit en sodium et en chlorure. Les demandeurs ont également constaté que ces souches ainsi définies par leur identité génétique pour le gène 18s, ont une identité génétique d'au moins 96% pour le gène d'actine (SEQ NO. 2), d'au moins 91% pour le gène de tubuline (SEQ NO. 3) et d'au moins 95% pour le gène EF1-alpha (SEQ NO. 4). Ces pourcentages d'identité sont repris dans le **Tableau 1(b)**.

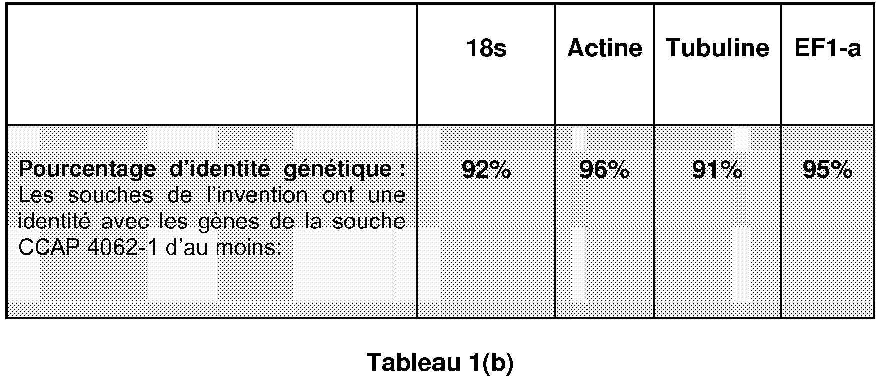

La **Figure 1** montre l'analyse phylogénétique qui a conduit à cette définition des souches concernées par l'invention.

Les souches de Thraustochytrides utilisées dans cette étude appartiennent au genre *Aurantiochytrium mangrovei, Schizochytrium sp* et *Schizochytrium aggregatum.*

Les séquences ont été alignées avec CLUSTAL W de Méga 5.1. Dans la Figure, les chiffres situés aux embranchements sont des valeurs de Bootstrap.

Une valeur de Bootstrap de 70% est considérée comme la limite en dessous de laquelle il ne faut pas descendre pour que l'embranchement entre deux groupes reste significatif. Selon Hillis D.M. et Bull J.J. dans leur article "An empirical test of bootstrapping as a method for assessing confidence in phylogenetic analysis" [(1993) Systematic Biology Vol. 42, pp. 182-192], des proportions Bootstrap de plus de 70% correspondent généralement à une probabilité d'au moins 95% que le clade (groupes) correspondant soit réel.

Dans la **Figure 1****,** cela signifie que la différence entre les groupes *A.mangrovei* et *Schizochytrium sp.* est significative car le nœud séparant les deux groupes a une valeur de 100%, et que nous sommes donc en présence de deux genres différents. Ces deux groupes sont eux-mêmes très éloignés du groupe des *Schizochytrium aggregatum,* comme cela avait déjà été démontré par Yokoyama et Honda [(2007), Taxonomic rearrangement of the genus Schizochytrium sensu lato based on morphology, chemotaxonomic characteristics, and 18S rRNA gene phylogeny (Thraustochytriaceae, Labyrinthulomycetes) : emendation for Schizochytrium and erection of Aurantiochytrium and Oblongichytrium gen. Nov.; Mycoscience Vol. 48, pp. 99-211].

Dans la **Figure 1**, les souches qui sont concernées par l'invention sont celles du premier groupe en haut de la Figure et ont le nom *Aurantiochytrium mangrovei.*

On peut ainsi citer comme exemples des souches qui sont concernées par l'invention, les souches *Aurantiochytrium sp.* SD116 (JX863672), *Aurantiochytrium limacinum* (AB022107), *Aurantiochytrium mangrovei* (DQ367049), *Aurantiochytrium limacinum* SL1101 (JN986842), *Aurantiochytrium limacinum* (JN986842), *Aurantiochytrium sp.* LY2012 (JX847370), *Aurantiochytrium limacinum* (HM042909) et *Aurantiochytrium sp.* BL10 (FJ821477), dans la classe phylogénétique classée comme identifiée par les inventeurs. Les numéros entre parenthèses sont des numéros d'accession.

En effet, chacune de ces souches présente un pourcentage d'identité aux séquences de la souche CCAP 4062/1 respectivement d'au moins 92%, 96%, 91% et 94% aux séquences SEQ NO. 1, SEQ NO. 2, SEQ NO. 3 et SEQ NO. 4.

Par exemple, des souches du genre *Aurantiochytrium* ayant une identité génétique de 92%, 93%, 94%, 95%, 96%, 97%, 98%, 99% ou 100% à la séquence SEQ NO. 1 sont concernées par l'invention. Ces souches ont également une identité génétique de 96%, 97%, 98%, 99% ou 100% à la séquence SEQ NO. 2, une identité génétique de 91%, 92%, 93%, 94%, 95%, 96%, 97%, 98%, 99% ou 100% à la séquence SEQ NO. 3 et une identité génétique de 95%, 96%, 97%, 98%, 99% ou 100% à la séquence SEQ NO. 4.

Le Tableau **1(a)** montre en détail la comparaison des identités génétiques des souches concernées, ou non, par l'invention, avec la souche CCAP 4062/1.

On note que ni la souche *Schizochytrium sp* ATCC 20888 (DQ367050), ni la souche FCC1412 *Schizochytrium sp.* SEK 209 (AB290574) n'appartiennent aux souches concernées par l'invention. Les valeurs d'identité à la SEQ NO. 1 pour ces souches sont de 91%.

Ces souches ont la capacité de croître à haute densité dans un milieu de culture chimiquement défini (c'est à dire en absence d'extrait de levure ou des autres extraits de protéines) ayant de très faibles quantités de chlorure de sodium. Ce milieu est caractérisé par le fait qu'il comporte moins de 3,5 g/L, de préférence moins de 1 g/L, plus préférentiellement, moins de 10 mg/L d'ions de sodium et moins de 1 g/L, de préférence moins de 0,5 g/L, plus préférentiellement, moins de 0,2 g/L d'ions de chlorure.

Dans **l'Exemple 1**, les demandeurs ont effectué des tests de croissance en Erlen avec des souches selon l'invention, ainsi que des souches de comparaison non concernées par l'invention, dans un tel milieu de culture (voir le **Tableau 2(a)** pour le milieu de culture principal). Les cultures ont été effectuées en présence d'azote minéral (NH₄)₂SO₄, et de glucose comme source de carbone, et sans apport d'azote organique. Ainsi, la **Figure 2** illustre des résultats issus de ses expériences. Les résultats montrent que toutes les souches d*'Aurantiochytrium mangrovei* ont la capacité de pousser sur ce milieu, contrairement aux autres genres de Thraustochytrides testés, *Schizochytrium sp.* et *Schizochytrium aggregatum.*

On constate que les souches selon l'invention ont une identité génétique pour les gènes 18s, actine, tubuline et EF1-alpha respectivement d'au moins 92%, 96%, 91% et 95% de la souche FCC 1324.

Les souches concernées par l'invention sont également caractérisées par leur profil lipidique. La souche CCAP 4062/1 est prise comme exemple et représentative des souches selon l'invention.

Les **Figures 3(A)** et **3 (B)** montrent une comparaison des profils d'acides gras entre les souches de deux genres différents. Le genre selon l'invention, illustré par *Aurantiochytrium mangrovei,* dont les *Aurantiochytrium limacinum* font partie (trois premières lignes de chaque panneau) et le genre des *Schizochytrium* sp., qui regroupe les souches ATCC 20888, ainsi que les souches *Aurantiochytrium sp* SEK 217 et SEK 209 (trois dernières lignes de chaque panneau). Le profil lipidique du genre *Aurantiochytrium mangrovei* présente une majorité de DHA (supérieur à 80% des PUFA totaux) dans les conditions de culture selon un mode de réalisation de l'invention, décrit dans **l'Exemple 2.**

Dans la Figure **3(A)**, on voit que les souches ATCC 20888, *Aurantiochytrium sp* SEK 217 et SEK 209 ont un profil différent avec plus d'EPA par rapport aux souches selon l'invention.

Le DPA (n-6) représente à peu près 20% des PUFAs totaux avec des quantités mineures d'AA et d'EPA (voir **Figure 3(A)****).** D'autres souches qui ont une similarité génétique à la souche CCAP 4062/1 présentent également ce profil.

La Figure **3(B)** montre que les souches ATCC 20888, *Aurantiochytrium sp* SEK 217 et SEK 209 ont un profil d'acides gras saturés différent, avec moins d'acide palmitique et une quantité plus importante d'acide gras impair C15:0 et C17:0.

La **Figure 4(A)** montre les acides gras polyinsaturés (PUFAs) exprimés en pourcentages par rapport aux PUFAs totaux. La **Figure 4(B)** montre les acides gras saturés exprimés en pourcentages par rapport aux acides gras saturés totaux. Les cultures dont le pH a été régulé par le KOH ou le NH₄OH ont été identifiées respectivement par le sigle (KOH) ou (NH₄OH). Le profil reste quasiment inchangé selon les conditions de culture. Le DHA est l'acide gras polyinsaturé majoritaire, à presque 80% des acides insaturés totaux. C16:0 reste l'acide gras saturé majoritaire, à plus de 90% des acides saturés totaux, dans les conditions de culture selon un mode de réalisation de l'invention, décrit dans les **Exemples 3** et **4.**

Ainsi, les inventeurs ont défini les souches concernées par l'invention. Ces souches ont une capacité à croître à forte densité en milieu de culture chimiquement défini, sans ajout de quantités significatives de sodium ni de chlorure.

La culture de ces souches s'effectue en général en mode hétérotrophe.

Le milieu de culture chimiquement défini selon l'invention comprend une source de carbone, une source d'azote et des sels nécessaires à la croissance des microorganismes. L'homme du métier connaît bien les éléments nécessaires à la croissance de microorganismes dans un procédé de fermentation.

Selon un mode préféré de réalisation de l'invention, la source de carbone est une source chimiquement définie choisie parmi le glucose, le glycérol, de préférence le glucose.

La teneur en source de carbone dans le milieu de culture est avantageusement comprise entre 10 et 90 g/L, ou 10 et 75 g/L.

La source d'azote est avantageusement une source minérale ou organique chimiquement définie, à l'exclusion de toute matière organique complexe comprenant de l'azote, tel que les extraits de levure ou des mélanges d'extrait protéiques.

Préférentiellement, la source d'azote est un sel d'ammonium, en particulier le sulfate d'ammonium, et/ou un nitrate, en particulier le nitrate de potassium.

La teneur en source d'azote dans le milieu de culture est avantageusement comprise entre 1 et 10 g/L.

Le milieu de culture principal peut contenir tous les autres composants connus de l'homme de l'art pour cultiver des microalgues selon l'invention. Le milieu contient en général des sels inorganiques chimiquement définis, par exemple, des sels de métaux alcalins et alcalinoterreux, ainsi que des sels des autres métaux. On peut citer comme exemple des sels de Ca, Mg, K, Fe, Ni, Co, Cu, Mn, Mo ou Zn. Par exemple, on peut citer des sulfates tels que le sulfate de potassium, le sulfate de magnésium, le sulfate de fer, le sulfate d'ammonium, le sulfate de magnésium, ou le sulfate de cuivre, le sulfate de nickel, le sulfate de zinc. On peut également citer des phosphates tels que le phosphate acide de potassium, ou les carbonates tels que le carbonate de calcium. On peut également citer de chlorures tels que le chlorure de cobalt, le chlorure de manganèse, le chlorure de calcium. On peut également citer des d'oxydes de métaux alcalins. On peut également citer des sélénites et des molybdates tel que le molybdate de sodium et le sélénite de sodium. D'autres sels inorganiques utilisables sont, par exemple, les halogénures, tels que le bromure de potassium ou l'iodure de potassium.

Les teneurs des différents sels dans le milieu de culture dépendent des besoins des microorganismes pour leur croissance. Certains sels ne sont employés qu'en faibles quantités comme apport d'oligoéléments, comme les sels de zinc, de cobalt, de manganèse, de molybdène, de sélénium, de nickel, ou de cuivre.

Un milieu de culture particulièrement préféré pour la méthode de l'invention comprend, le cas échéant, à part d'autres éléments tels que, par exemple, des composants nutritifs, au moins un des sels choisi dans le groupe comprenant le sulfate de magnésium, le chlorure de calcium, le borate et le phosphate de potassium. Le(s) sel (s) est / sont ajoutés sans que la teneur totale en sel selon l'invention soit dépassée. Il est particulièrement préféré lorsque le sulfate de magnésium, le chlorure de calcium et le phosphate de potassium sont ajoutés au milieu.

Avantageusement, la teneur en ions est comprise dans les gammes détaillées dans le Tableau 2(a) (cf. tableau suivant).

| **Ingrédients** | **Concentration** | **Gammes de concentration** | | |
|---|---|---|---|---|
| ***Solution Principale*** | | ***Générale*** | ***Préférée*** | ***Plus préférée*** |
| KCI | g/L | 0-1 | 0,05-0,5 | 0,1-0,5 |
| H₃BO₃ | g/L | 0-0,5 | 0,01-0,3 | 0,1-0,2 |
| MgSO₄, 7H₂0 | g/L | 0,5-13 | 2-10 | 5-7 |
| CaCl₂, 2H₂0 | g/L | 0,1-1,1 | 0,2-0,9 | 0,3-0,6 |
| KNO₃ | g/L | 0-0,1 | 0,01-0,06 | 0,02-0,05 |
| KH₂PO₄, 7H2O | g/L | 0,1-1,5 | 0,2-1 | 0,3-0,7 |
| Na₂EDTA, 2H₂O | g/L | 0-0,01 | 0,001-0,005 | 0,002-0,004 |
| ZnSO₄.7H₂O | mg/L | 0,001-0,1 | 0,01-0,1 | 0,02-0,08 |
| CoCl₂.6H₂O | mg/L | 0,001-0,1 | 0,01-0,1 | 0,02-0,08 |
| MnCl₂.4H₂O | mg/L | 0,01-2 | 0,05-1 | 0,25-0,7 |
| Na₂MoO₄, 2H₂O | mg/L | 0,0001-1 | 0,0005-0,1 | 0,001-0,1 |
| Na₂SeO₃ | mg/L | 0-1 | 0,01-0,5, | 0,01-0,02 |
| NiSO₄.6H₂O | mg/L | 0,0001-50 | 0,5-5 | 0,001-0,006 |
| CuSO₄.5H₂0 | mg/L | 0,001-3 | 0,0025-1 | 0,005-0,02 |
| EDTA-Fe | mg/L | 1-100 | 10-50 | 20-40 |

### Carbone

| | | | | |
|---|---|---|---|---|
| Glucose | g/L | 10-90 | 20- 60 | 45-60 |

### Azote

| | | | | |
|---|---|---|---|---|
| (NH₄)₂SO₄ | g/L | 1-10 | 2-9 | 6-8 |

### Post Autoclave

### Vitamines

**Tableau 2(a) : Composition générale du milieu.**

| | | | | |
|---|---|---|---|---|
| Thiamine | mg/L | 0,1-100 | 1-50 | 5-10 |
| Vitamine B12 | mg/L | 0,01-100 | 0,025-5 | 0,1-2 |
| Panthothénate | mg/L | 0,1-100 | 0,1-25 | 1-10 |

Dans tous les cas, la nature et les proportions de ces sels dans le milieu de culture sont choisies de manière que la teneur en ions sodium soit de moins de 3,5 g/L, de préférence moins de 1 g/L, plus préférentiellement moins de 10 mg/L et que la teneur en ions chlorure soit de moins de 1 g/L, de préférence moins de 500 mg/L, plus préférentiellement d'environ 200 mg/L.

Selon un mode préférentiel de réalisation de l'invention, le milieu de culture comprend également des macro- ou micronutriments supplémentaires, tels que des acides aminés, purine, pyrimidine, des vitamines qui sont chimiquement définies, c'est à dire à l'exclusion des macro- ou micronutriments apportés dans le milieu de culture par des sources complexes, comme la levure. De manière générale, le milieu comporte des et d'autres composants médias connus à l'homme du métier. Les agents anti-moussants peuvent être ajoutés, si nécessaire. Toutefois le milieu ne contient pas de composants complexes qui ne peuvent pas être définis chimiquement.

Les vitamines sont avantageusement choisies parmi la thiamine, la vitamine B12, le panthothénate et leurs mélanges.

La teneur en vitamines dans le milieu de culture est avantageusement comprise entre les valeurs décrites dans le **Tableau 2(a).**

Selon un mode préférentiel de réalisation de l'invention, le milieu de culture chimiquement défini consiste en un mélange d'une source de carbone chimiquement définie, d'une source d'azote chimiquement définie, de sels et de vitamines chimiquement définis.

On notera que ce milieu de culture est particulièrement approprié pour une culture en fermenteur, avantageusement dans des fermenteurs d'au moins 1000 litres, notamment en mode discontinu dit "batch", en mode semi-continu dit "fed batch" ou en mode continu.

Un exemple d'un milieu de culture selon un mode de réalisation de l'invention est défini tel que dans le **Tableau 2(b)** ci-dessous.

### Solution Principale

| | | |
|---|---|---|
| KCl | 0,30 | g/L |
| H₃BO₃ | 0,0175 | g/L |
| MgSO₄, 7H₂0 | 5,25 | g/L |
| CaCl₂, 2H₂0 | 0,45 | g/L |
| KNO₃ | 0,04 | g/L |
| KH₂PO₄, 7H2O | 0,309 | g/L |
| Na₂EDTA, 2H₂O | 0,0020 | g/L |
| ZnSO₄.7H₂O | 0,08 | mg/L |
| CoCl₂.6H₂O | 0,08 | mg/L |
| MnCl₂.4H₂O | 0,40 | mg/L |
| Na₂MoO₄, 2H₂O | 0,00100 | mg/L |
| Na₂SeO₃ | 0,00173 | mg/L |
| NiSO₄.6H₂O | 0,00500 | mg/L |
| CuSO₄.5H₂0 | 0,015 | mg/L |
| EDTA-Fe | 20 | mg/L |

### Carbone

| | | |
|---|---|---|
| Glucose | 50 | g/L |

### Azote

| | | |
|---|---|---|
| (NH₄)₂SO₄ | 8 | g/L |

### Post Autoclave

### Vitamines

**Tableau 2(b) : Exemple de Milieu de culture.**

| | | |
|---|---|---|
| Thiamine | 6 | mg/L |
| Vitamine B12 | 0,15 | mg/L |
| Panthothénate | 4,0 | mg/L |

Un exemple d'un milieu de culture selon un mode de réalisation de l'invention est défini tel que dans le **Tableau 2(c)** ci-dessous. Ce milieu nommé FCC-M comporte 0,00038 g/l de sodium (Na⁺) et 0,437 g/l de chlorure (Cl⁻). Le milieu ne contient pas de NaCl ajouté.

### Solution Principale

| **Ingrédients** | **Concentration** |
|---|---|
| | g/L |
| KCl | 0,36 |
| H₃BO₃ | 0,175 |
| MgSO₄, 7H₂0 | 6,750 |
| CaCl₂, 2H₂0 | 0,55 |
| KNO₃ | 0,04667 |
| KH₂PO₄,7H2O | 0,30940 |
| Na₂EDTA, 2H₂O | 0,003094 |
| ZnSO₄.7H₂O | 0,000073 |
| CoCl₂.6H₂O | 0,000016 |
| MnCl₂.4H₂O | 0,00054 |
| Na₂MoO₄, 2H₂O | 0,00000148 |
| Na₂SeO₃ | 0,00000173 |
| NiSO₄.6H₂O | 0,00000298 |
| CuSO₄.5H₂0 | 0,0000098 |
| EDTA-Fe | 0,03 |

| | |
|---|---|
| ***Carbone*** | g/L |
| Glucose | 55 |

| | |
|---|---|
| ***Azote*** | g/L |
| (NH₄)₂SO₄ | 7 |

### Post Autoclave

**Tableau 2(c): Milieu de culture FCC-M.**

| ***Vitamines*** | g/L |
|---|---|
| Thiamine | 0,008 |
| Vitamine B12 | 0,00013 |
| Panthothénate | 0,0027 |

De manière classique, lors de la culture des souches selon l'invention en bioréacteur ou en fermenteur, en conditions hétérotrophes ou mixotrophes, le milieu de culture de base est supplémenté par une solution d'ajout pour maintenir la croissance des microalgues. Le substrat carboné, par exemple du glucose, peut être également ajouté (solution d'ajout 2). L'homme du métier sait déterminer la concentration de chacun des éléments de la solution d'ajout. Par exemple, les teneurs des éléments à titre d'exemple sont indiquées dans le **Tableau 3 (a)** ci-dessous.

**Tableau 3 (a) : Exemple des solutions d'ajout 1 et 2 pour une culture en fermenteur ou en bioréacteur.**

| ***Solution d'ajout 1*** | g/L |
|---|---|
| K₂SO₄ | 0 - 35 |
| MgSO₄, 7H₂0 | 0-30 |
| KH₂PO₄,7H2O | 50-70 |
| FeSO₄, 7H₂0 | 0-1 |
| (NH₄)₂SO₄ * | 120-150 |
| MnCl₂ 4H₂O | 0,10-0,2 |
| ZnSO₄.7H₂O | 0,1-0,2 |
| CoCl₂.6H₂O | 0,001-0,002 |
| Na₂MoO₄, 2H₂O | 0,001-0,002 |
| CuSO₄, 5H₂O | 0,05-0,2 |
| NiSO₄, 6H₂O | 0,05-0,2 |
| Na₂EDTA, 2H₂O | 1-3 |
| Thiamine | 1-2 |
| Vitamine B12 | 0, 005-0,02 |
| Panthothénate | 0,05-0,3 |

| ***Solution d'ajout 2*** | g/L |
|---|---|
| Glucose | 150- 850 |
| KH₂PO₄ | 4 - 7 |
| (NH₄)₂SO₄ * | 20-40 |

Un exemple de telles solutions d'ajout est donné dans le **Tableau 3(b).**

**Tableau 3(b): Exemple des solutions d'ajout 1 et 2 pour une culture en fermenteur ou en bioréacteur.**

| ***Solution d'ajout 1*** | **Concentration** |
|---|---|
| | g/L |
| K₂SO₄ | 31,9 |
| MgSO₄, 7H₂0 | 25,8 |
| KH₂PO₄,7H2O | 61,38 |
| FeSO₄, 7H₂0 | 0,61 |
| (NH₄)₂SO₄ * | 138,24 |
| MnCl₂ 4H₂O | 0,165 |
| ZnSO₄.7H₂O | 0,165 |
| CoCl₂.6H₂O | 0,0016 |
| Na₂MoO₄, 2H₂O | 0,0016 |
| CuSO₄, 5H₂O | 0,11 |
| NiSO₄, 6H₂O | 0,086 |
| Na₂EDTA, 2H₂O | 1,81 |
| Thiamine | 0,49 |
| Vitamine B12 | 0,008 |
| Panthothénate | 0,1656 |

| ***Solution d'ajout 2*** | g/L |
|---|---|
| Glucose | 750 |
| KH₂PO₄ | 6,4 |
| (NH₄)₂SO₄ * | 34 |

Selon un mode de réalisation de l'invention, quand la culture est effectuée en fermenteur ou en bioréacteur, typiquement, après l'ajout des solutions d'ajout pour maintenir la croissance des cellules, la concentration finale de Na⁺ est à environ 10 mg/L, de préférence moins de 6 mg/L, et la concentration finale de Cl⁻ est à environ 250 mg/L, de préférence moins de 200 mg/L.

Selon un autre mode de réalisation de l'invention, quand la culture est effectuée en Erlen, la concentration de Na⁺ est à moins de 5 mg/L, de préférence moins de 2 mg/L et plus préférentiellement moins de 1 mg/L et la concentration de Cl⁻ est à environ 1 g/L, de préférence moins de 0,750 g/L et plus préférentiellement moins de 0,5 g/L.

De manière générale, en cours de culture, des ajouts d'un substrat carboné organique sont effectués (voir, par exemple, la solution d'ajout 2 **Tableau 3(a ou b)),** afin de permettre aux cellules d'accumuler une concentration importante de lipides. Du substrat additionnel (solution d'ajout 2) est ajouté au milieu de culture pendant le procédé de culture pour maintenir une concentration suffisante. Ce substrat carboné organique comprend préférentiellement, sous forme pure ou en mélange : du glucose, des dérivés de cellulose, du saccharose et/ou du glycérol. La concentration en substrat organique est généralement entre 200 mM et 500 mM.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en hétérotrophie ou en mixotrophie des protistes selon l'invention.

Lors de la culture, le pH se situe entre 4 et 8, la température entre 20 et 30°C et la concentration d'oxygène dissous est typiquement régulée entre 5% et 30%.

Ce procédé a pour avantage d'augmenter le rendement en biomasse obtenu de la culture. Il a aussi pour avantage d'enrichir les protistes ainsi cultivés en acides gras polyinsaturés, plus particulièrement en acide docosahexaénoïque (DHA).

Selon un mode de réalisation de l'invention, une préculture est réalisée dans un milieu de culture ayant une faible quantité de NaCl, tel que le milieu FCC-M **(Tableau 2(c))** contenant, par exemple, de l'extrait de levure comme source d'azote, et du glucose, par exemple, comme source de carbone. Après un temps de d'incubation, par exemple 48 heures, les cellules sont centrifugées et le culot de cellules rincé dans un milieu de culture ayant une faible quantité de NaCl, tel que le FCC-M, par exemple, ne contenant ni extrait de levure, ni aucune autre source d'azote minérale ou organique. Cette opération a pour but d'éviter tout apport de Na⁺ dans la culture principale via la présence d'extrait de levure dans la préculture, correspondant généralement à 1/100 (v/v) du volume de culture de la solution principale.

Les souches *Aurantiochytrium* isolées selon l'invention permettent de produire des quantités significatives de biomasse ainsi que de lipides, les lipides étant riches en DHA. En effet, le procédé de l'invention en conditions hétérotrophe ou mixotrophe permet d'obtenir un rendement en biomasse supérieur à 100 g/L, de préférence supérieur à 120 g/L, cette biomasse ayant 50% à 60% de lipides par rapport au poids de la matière sèche. Le DHA peut représenter plus de 15%, ou plus de 20%, ou plus de 30% des acides gras totaux contenus dans les protistes. Les protistes, selon un mode de réalisation de l'invention, peuvent avoir ainsi une productivité (quantité de produit d'intérêt produit, par litre de culture, par heure) de DHA d'au moins 0,1 g/L/h, de préférence d'au moins 0,2 g/L/h, et plus préférentiellement d'au moins 0,3 g/L/h.

Le procédé selon l'invention comporte en outre les étapes suivantes :
a) la culture, en conditions hétérotrophes, d'une ou plusieurs souches de *Labyrinthulomycete,* en particulier du genre *Aurantiochytrium* ayant une identité génétique d'au moins 92% à la séquence SEQ NO. 1 dans un milieu de culture chimiquement défini ayant moins de 1 g/L, de préférence, moins de 10 mg/L sodium (Na⁺) et moins de 1 g/L, de préférence, moins de 200 mg/L de chlorure (Cl⁻),
b) une étape de maintien de ladite culture sur plusieurs générations,
c) une étape de récupération de la biomasse ainsi cultivée.

Par étape de récupération, on entend plus particulièrement l'isolement de la ou des souches dont le nombre de cellules s'est accru le plus au cours desdites générations.

Le procédé peut également comprendre les étapes additionnelles :
d) une étape de récupération des lipides des souches, et éventuellement,
e) l'extraction du DHA (acide docosahexaénoïque) des lipides récupérés.

Les souches de l'étape (a) peuvent également avoir une identité génétique d'au moins 96% à la séquence SEQ NO. 2, et/ou une identité génétique d'au moins 91% à la séquence SEQ NO. 3 et/ou une identité génétique d'au moins 95% à la séquence SEQ NO. 4.

Le procédé de culture selon l'invention permet d'effectuer les cultures de ces souches *d'Aurantiochytrium* dans des milieux de cultures avec un taux réduit en sodium et en chlorure, sans perdre la productivité et de hauts rendements en biomasse, en lipides et en particulier en DHA. Ainsi, on évite la dégradation des fermenteurs et autres équipements en acier inoxydable, utilisés pour la culture des cellules et le traitement de la biomasse résultante, ainsi que la dégradation précoce par corrosion des outils de production et traitement de la biomasse (« Down-stream Processing » ou « DSP » en anglais).

L'invention concerne également la mise au point d'un milieu de culture permettant la production à haute densité cellulaire de souches selon l'invention riches en DHA. Le milieu est chimiquement défini à teneur réduite en ions de sodium (Na⁺) et de chlorure (Cl⁻). La concentration de Na⁺ est généralement à moins de 100 mg/L, de préférence moins de 50 mg/L et plus préférentiellement moins de 6 mg/L, et la concentration de Cl⁻ est de préférence à moins de 0,5 g/L, et plus préférentiellement moins de 200 mg/L. Selon un mode de réalisation de l'invention, la culture des souches concernées par l'invention est réalisée dans un milieu de culture ayant moins de 0,5 g/L NaCl, moins de 6 mg/L d'ions de sodium et moins de 200 mg/L d'ions de chlorure.

Selon un mode de réalisation, le milieu de culture est le FCC-M (Tableau 2(c)). Si la culture est effectuée en fermenteur ou en bioréacteur, des solutions d'ajout seront souhaitables, tel que décrit plus haut (voir par exemple le **Tableau 3(a ou b)).**

Puisque les milieux de culture selon l'invention sont chimiquement définis, ils ne comportent ni agents de croissance, tels que les extraits de levure ou des peptones qui, eux aussi, comportent des quantités de chlorure de sodium, ni agents régulateurs osmotiques, tels que le mannitol ou le sorbitol. Ainsi, en l'absence de ces agents, la biomasse et les lipides issus de la culture peuvent être utilisés pour des produits alimentaires (ou pharmaceutiques) sans les nombreuses étapes de DSP nécessaires pour, soit caractériser le contenu des produits finaux, soit éliminer ces agents ajoutés non souhaités dans les produits finaux. Ainsi on évite des surcoûts associés à ces étapes supplémentaires. De même, les coproduits obtenus après extraction de l'huile peuvent être utilisés pour l'alimentation animale sous forme de tourteaux par exemple.

Un autre avantage du procédé de l'invention et du milieu de l'invention est que les effluents issus des cultures ne contiennent pas d'agents nécessitant des étapes de traitement supplémentaires qui entraînent des coûts supplémentaires et qui réduisent ainsi la rentabilité de la production.

La méthode et le milieu de l'invention permettent non seulement d'optimiser la production de la biomasse obtenue de la culture, tout en évitant l'utilisation des ions de sodium et de chlorure et les problèmes de surcoûts associés, mais aussi d'enrichir les organismes ainsi cultivés en acides gras polyinsaturés.

De préférence, les souches sont cultivées selon les procédés visés précédemment, puis récupérées pour en extraire le contenu lipidique, en particulier les lipides dont le DHA. Les méthodes d'extraction sélective des lipides, dont le DHA, sont connues de l'homme du métier et sont, par exemple, décrites par [Bligh, E.G. et Dyer, W.J. (1959) [A rapid method of total lipid extraction and purification, Can. J. Biochem. Physiol., 37:911-917].

Les souches selon un mode de réalisation de l'invention peuvent avoir ainsi une productivité de DHA d'au moins 0,1 g/L/h, de préférence d'au moins 0,2 g/L/h, et plus préférentiellement d'au moins 0,3 g/L/h.

L'invention concerne également la utilisation de tout ou partie de la biomasse et/ou des coproduits obtenus à partir du procédé d'invention en tant que produit, ingrédient dans un produit pour l'alimentation humaine ou comme matière première pour l'alimentation animale notamment l'aquaculture.

L'invention concerne également l'utilisation du milieu de culture selon des modes de réalisation, décrits dans ce texte, en particulier le milieu de culture nommé FCC-M, pour la culture de protistes pour la production de lipides et de pigments.

### Exemple 1

### Tests de croissance en Erlen :

Les souches listées dans la Figure 2 ont été préalablement cultivées dans un milieu contenant du sel de mer reconstitué, Instant Ocean^{®} à 15 g/l pendant deux jours, puis centrifugées et lavées une fois avec la solution FCC-M (Tableau 2(c)), avant d'être inoculées (1/1000 v/v) dans les Erlens contenant 50 ml de milieu FCC-M. Les densités optiques des cultures cellulaires ont été mesurées après 3 jours d'incubation à 26°C et sous agitation (220 rpm).

### Exemple 2

### Profils d'acides gras de cellules ayant poussé en Erlen :

Les souches listées dans la Figure 2 ont été préalablement cultivées dans un milieu contenant du sel de mer reconstitué, Instant Ocean^{®} à 15 g/l pendant deux jours, puis centrifugées et lavées une fois avec la solution FCC-M (Tableau 2(c)), avant d'être inoculées (1/1000 v/v) dans les Erlens contenant 50 ml de milieu FCC-M, dans lequel le sulfate d'ammonium a été substitué par de l'extrait de levure (4 g/L). Le profil d'acides gras (FAMEs) a été déterminé à partir de cultures cellulaires incubées pendant 3 jours à 26°C et sous agitation (220 rpm).

### Exemple 3

### Tests de croissance et production de DHA en bioréacteur :

Les cultures *d'Aurantiochytrium* ont été réalisées dans des fermenteurs (bioréacteurs) de 1 à 2 L utiles avec automates dédiés et supervision par station informatique. Le système a été régulé en pH via l'ajout de base (NaOH, ou KOH) et/ou d'acide (solution d'acide sulfurique). La température de culture a été fixée à 26°C. L'agitation a été réalisée grâce à 3 mobiles d'agitation placés sur l'arbre selon la configuration de Rushton (hélices tripales à pompage descendant). La pression d'oxygène dissous a été régulée dans le milieu tout au long de la culture, par la vitesse d'agitation (250 - 1200 t/min), le débit d'air (0,25 - 1 vvm), voire le débit d'oxygène (0,1 - 0,5 vvm). Les paramètres de régulation, intégrés dans l'automate de supervision, ont permis de maintenir une pO₂ constante comprise entre 5% et 30%. Le temps de culture a été compris entre 50 et 200 heures, de préférence entre 65 et 96 heures, par exemple 72 heures.

Une préculture a été réalisée sur table d'agitation (140 t/min) en enceinte thermostatée (26°C), en milieu FCC-M, contenant 4 g d'extrait de levure comme source d'azote et de 30 g de glucose comme source de carbone. Après 48 heures d'incubation, les cellules ont été centrifugées pendant 5 minutes à 3000 g et le culot de cellules a été rincé avec du milieu FCC-M ne contenant ni extrait de levure, ni aucune autre source d'azote minérale ou organique. En cours de culture, 3 ajouts de solution d'ajout 1 ont été effectuées, ainsi que des ajouts de la solution 2 afin de maintenir des concentrations en glucose comprises entre 200 mM et 500 mM.

### Suivi des cultures :

La concentration en biomasse totale a été suivie par mesure de la masse sèche (filtration sur filtre GF/F, Whatman, puis séchage en étuve, à 105°C, pendant 24 h minimum avant pesée).

Les analyses en contenus lipidiques totaux et des FAMEs ont été réalisées selon les méthodes classiquement décrites dans la littérature [Folch J, et al., A simple method for the isolation and purification of total lipides from animal tissues. J Biol Chem. 1957 May; 226(1):497-509].

### Exemple 4

### Culture en Fermenteur :

Les cultures *d'Aurantiochytrium* ont été réalisées dans des fermenteurs, de manière similaire à celle décrite dans **l'Exemple 3.** La modification de la procédure a été réalisée sur le mode de régulation du pH par ajout d'ammoniaque (NH₄OH) pour éviter l'apport important de Na⁺ ou de K⁺, lié à la régulation du pH par NaOH ou la KOH, qui aurait pu être gênant pour la valorisation des coproduits pour l'alimentation animale. Une partie de l'azote nécessaire à la culture des cellules étant apportée via la régulation du pH par l'ammoniaque (NH₄OH), il n'était donc plus nécessaire d'inclure le (NH₄)₂SO₄ dans la composition de la solution d'ajout 1.

Le Tableau 3 indique les résultats de cet exemple :

| | **Masse Sèche g/l** | **Productivité en MS (g/L/h)** | **MG/MS%** | **DHA/MG %** | **DHA g/L** |
|---|---|---|---|---|---|
| CCAP 4062-1 FCC-M (KOH) | 129.2 | 1.83 | 40.75 | 31.92 | 16.81 |
| CCAP 4062-1 FCC-M | 127.9 | 1.67 | 50 | 31.6 | 20.85 |
| CCAP 4062-1 FCC-M (NH₄OH) | 120 | 1.566 | 35.09 | 38.01 | 16.01 |
| CCAP 4062-1 Instant Ocean^{®} | 131.5 | 1.85 | 49.9 | 23.00 | 15.09 |

## Revendications

1. Procédé de production de acide docosahexaénoïque (DHA) comprenant l'étape suivante :
la culture, en conditions hétérotrophes ou mixotrophes, d'une ou plusieurs souches du genre *Aurantiochytrium* dans un milieu de culture ayant moins de 3,5 g/L d'ions de sodium,
**caractérisé en ce que** les souches du genre *Aurantiochytrium* ont une identité génétique d'au moins 92% à la séquence SEQ NO. 1 et
**en ce que** le milieu de culture est un milieu chimiquement défini ayant moins de 3,5 g/L d'ions de sodium et moins de 1 g/L d'ions de chlorure et ayant de 200 mM à 500 mM de substrat carboné organique.

2. Procédé de culture selon la revendication 1, **caractérisé en ce que** la ou les souches du genre *Aurantiochytrium* a ou ont également une identité génétique d'au moins 96 % à la séquence SEQ NO. 2, et/ou d'au moins 91 % à la séquence SEQ NO. 3 et/ou d'au moins 95% à la séquence SEQ NO. 4.

3. Procédé de culture selon la revendication 1 ou 2, **caractérisé en ce que** le milieu de culture a moins de 1 g/L, de préférence moins de 100 mg/L, plus préférentiellement moins de 6 mg/L d'ions de sodium.

4. Procédé selon l'une quelconque des revendications 1 à 3, **caractérisé en ce que** le milieu de culture a moins de 500 mg/L, préférentiellement moins de 200 mg/L d'ions de chlorure.

5. Procédé selon l'une quelconque des revendications 1 à 4, **caractérisé en ce que** le milieu ne comporte pas d'agent régulateur de la pression osmotique, tel que le mannitol, le sorbitol, le polyéthylène glycol et le saccharose.

6. Procédé selon l'une quelconque des revendications 1 à 5 comprenant en outre les étapes :
b) de maintien de ladite culture sur plusieurs générations,
c) de récupération de la biomasse ainsi cultivée,
d) de récupération des lipides des souches, et éventuellement,
e) l'extraction du DHA (acide docosahexaénoïque).

7. Procédé selon l'une quelconque des revendications 1 à 6, **caractérisé en ce que** le milieu de culture consiste en :
| Ingrédients | Concentration |
|---|---|
| KCl | 0,05-0,5 g/L |
| H₃BO₃ | 0,01-0,3 g/L |
| MgSO₄, 7H₂0 | 2-10 g/L |
| CaCl₂, 2H₂0 | 0,2-0,9 g/L |
| KNO₃ | 0,01-0,06 g/L |
| KH₂PO₄,7H2O | 0,2-1 g/L |
| Na₂EDTA, 2H₂O | 0,001-0,005 g/L |
| ZnSO₄.7H₂O | 0,01-0,1 mg/L |
| CoCl₂.6H₂O | 0,01-0,1 mg/L |
| MnCl₂.4H₂O | 0,05-1 mg/L |
| Na₂MoO₄, 2H₂O | 0,0005-0,1 mg/L |
| Na₂SeO₃ | 0,01-0,5 mg/L |
| NiSO₄.6H₂O | 0,5-5 mg/L |
| CuSO₄.5H₂0 | 0,0025-1 mg/L |
| EDTA-Fe | 10-50 mg/L |
| Glucose | 20-60 g/L |
| (NH₄)₂SO₄ | 2-9 g/L |
| Thiamine | 1-50 mg/L |
| Vitamine B12 | 0,025-5 mg/L |
| Panthothénate | 0,1-25 mg/L |

8. Procédé selon la revendication 7, **caractérisé en ce que** la biomasse issue de l'étape b) représente au moins 100 g/L de matière sèche.

9. Procédé selon la revendication 7 ou 8, **caractérisé en ce que** la concentration de DHA à la fin de l'étape b) représente au moins 15 g/L.

10. Procédé selon l'une quelconque des revendications 7 à 9, **caractérisé en ce que** le DHA contenu dans la biomasse à l'issue de l'étape b) représente plus de 30% des lipides totaux.

11. Procédé selon l'une quelconque des revendications 1 à 10, **caractérisé en ce que** ledit organisme du genre *Aurantiochytrium* correspond à la souche FCC 1324, déposée auprès de la CCAP (Culture Collection of Algae and Protozoa), sous le numéro d'accession CCAP 4062/1.

12. Utilisation de tout ou partie de la biomasse obtenue à partir du procédé selon l'une quelconque des revendications 1 à 11 en tant que produit, ingrédient dans un produit pour l'alimentation humaine ou comme matière première pour l'alimentation animale notamment l'aquaculture.

13. Milieu de culture **caractérisé en ce qu'**il consiste en :
| Ingrédients | Concentration |
|---|---|
| KCl | 0,05-0,5 g/L |
| H₃BO₃ | 0,01-0,3 g/L |
| MgSO₄, 7H₂0 | 2-10 g/L |
| CaCl₂, 2H₂0 | 0,2-0,9 g/L |
| KNO₃ | 0,01-0,06 g/L |
| KH₂PO₄,7H2O | 0,2-1 g/L |
| Na₂EDTA, 2H₂O | 0,001-0,005 g/L |
| ZnSO₄.7H₂O | 0,01-0,1 mg/L |
| CoCl₂.6H₂O | 0,01-0,1 mg/L |
| MnCl₂.4H₂O | 0,05-1 mg/L |
| Na₂MoO₄, 2H₂O | 0,0005-0,1 mg/L |
| Na₂SeO₃ | 0,01-0,5 mg/L |
| NiSO₄.6H₂O | 0,5-5 mg/L |
| CuSO₄.5H₂0 | 0,0025-1 mg/L |
| EDTA-Fe | 10-50 mg/L |
| Glucose | 20-60 g/L |
| (NH₄)₂SO₄ | 2-9 g/L |
| Thiamine | 1-50 mg/L |
| Vitamine B12 | 0,025-5 mg/L |
| Panthothénate | 0,1-25 mg/L |

14. Utilisation du milieu de culture selon la revendication 13 pour la culture de protistes pour la production de lipides et de pigments.

15. Organisme du genre *Aurantiochytrium* correspondant à la souche FCC 1324, déposée auprès de la CCAP (Culture Collection of Algae and Protozoa), sous le numéro d'accession CCAP 4062/1.

## Patentansprüche

1. Verfahren zur Herstellung von Docosahexaensäure (DHA), umfassend den folgenden Schritt:
Kultivieren eines oder mehrerer Stämme der Gattung *Aurantiochytrium* unter heterotrophen oder mixotrophen Bedingungen in einem Kulturmedium mit weniger als 3,5 g/l Natriumionen,
**dadurch gekennzeichnet, dass** die Stämme der Gattung *Aurantiochytrium* eine genetische Identität von mindestens 92 % in der Sequenz SEQ NO. 1 aufweisen und
dass das Kulturmedium ein chemisch definiertes Medium mit weniger als 3,5 g/l Natriumionen und weniger als 1 g/l Chloridionen und mit 200 mM bis 500 mM organischem Kohlenstoffsubstrat ist.

2. Kulturverfahren nach Anspruch 1, **dadurch gekennzeichnet, dass** der oder die Stämme der Gattung *Aurantiochytrium* eine genetische Identität von mindestens 96 % in der Sequenz SEQ NO. 2 und/oder von mindestens 91 % in der Sequenz SEQ NO. 3 und/oder von mindestens 95 % in der Sequenz SEQ NO. 4 aufweisen.

3. Kulturverfahren nach Anspruch 1 oder 2, **dadurch gekennzeichnet, dass** das Kulturmedium weniger als 1 g/l, vorzugsweise weniger als 100 mg/l, bevorzugter weniger als 6 mg/l Natriumionen aufweist.

4. Verfahren nach einem der Ansprüche 1 bis 3, **dadurch gekennzeichnet, dass** das Kulturmedium weniger als 500 mg/l, vorzugsweise weniger als 200 mg/l Chloridionen aufweist.

5. Verfahren nach einem der Ansprüche 1 bis 4, **dadurch gekennzeichnet, dass** das Medium kein osmotisches Druckregelmittel wie Mannitol, Sorbitol, Polyethylenglykol und Saccharose enthält.

6. Verfahren nach einem der Ansprüche 1 bis 5, ferner umfassend die Schritte:
b) Aufrechterhalten dieser Kultur über mehrere Generationen hinweg,
c) Rückgewinnen der so gezüchteten Biomasse,
d) Rückgewinnen der Lipide aus den Stämmen und gegebenenfalls
e) Extrahieren von DHA (Docosahexaensäure).

7. Verfahren nach einem der Ansprüche 1 bis 6, **dadurch gekennzeichnet, dass** das Kulturmedium aus Folgendem besteht:
| Bestandteile | Konzentration |
|---|---|
| KCl | 0,05-0,5 g/l |
| H₃BO₃ | 0,01-0,3 g/l |
| MgSO₄, 7H₂0 | 2-10 g/l |
| CaCl₂, 2H₂0 | 0,2-0,9 g/l |
| KNO₃ | 0,01-0,06 g/l |
| KH₂PO₄, 7H2O | 0,2-1 g/l |
| Na₂EDTA, 2H₂O | 0,001-0,005 g/l |
| ZnSO₄.7H₂O | 0,01-0,1 mg/l |
| CoCl₂.6H₂O | 0,01-0,1 mg/l |
| MnCl₂.4H₂O | 0,05-1 mg/l |
| Na₂MoO₄, 2H₂O | 0,0005-0,1 mg/l |
| Na₂SeO₃ | 0,01-0,5 mg/l |
| NiSO₄.6H₂O | 0,5-5 mg/l |
| CuSO₄.5H₂0 | 0,0025-1 mg/l |
| EDTA-Fe | 10-50 mg/l |
| Glukose | 20-60 g/l |
| (NH₄)₂SO₄ | 2-9 g/l |
| Thiamin | 1-50 mg/l |
| Vitamin B12 | 0,025-5 mg/l |
| Panthothenat | 0,1-25 mg/l |

8. Verfahren nach Anspruch 7, **dadurch gekennzeichnet, dass** die aus Schritt b) stammende Biomasse mindestens 100 g/l Trockenmasse ausmacht.

9. Verfahren nach Anspruch 7 oder 8, **dadurch gekennzeichnet, dass** die DHA-Konzentration an dem Ende von Schritt b) mindestens 15 g/l beträgt.

10. Verfahren nach einem der Ansprüche 7 bis 9, **dadurch gekennzeichnet, dass** die in der Biomasse nach Schritt b) enthaltene DHA mehr als 30 % der Gesamtlipide ausmacht.

11. Verfahren nach einem der Ansprüche 1 bis 10, **dadurch gekennzeichnet, dass** der Organismus der Gattung *Aurantiochytrium* dem Stamm FCC 1324 entspricht, der bei der CCAP (Culture Collection of Algae and Protozoa) unter der Zugangsnummer CCAP 4062/1 hinterlegt ist.

12. Verwendung der gesamten oder eines Teils der aus dem Verfahren nach einem der Ansprüche 1 bis 11 gewonnenen Biomasse als Produkt, als Bestandteil in einem Produkt für die menschliche Ernährung oder als Rohstoff für die Tierernährung, insbesondere für die Aquakultur.

13. Kulturmedium, das **dadurch gekennzeichnet ist, dass** es aus Folgendem besteht:
| Bestandteile | Konzentration |
|---|---|
| KCl | 0,05-0,5 g/l |
| H₃BO₃ | 0,01-0,3 g/l |
| MgSO₄, 7H₂0 | 2-10 g/l |
| CaCl₂, 2H₂0 | 0,2-0,9 g/l |
| KNO₃ | 0,01-0,06 g/l |
| KH₂PO₄, 7H2O | 0,2-1 g/l |
| Na₂EDTA, 2H₂O | 0,001-0,005 g/l |
| ZnSO₄.7H₂O | 0,01-0,1 mg/l |
| CoCl₂.6H₂O | 0,01-0,1 mg/l |
| MnCl₂.4H₂O | 0,05-1 mg/l |
| Na₂MoO₄, 2H₂O | 0,0005-0,1 mg/l |
| Na₂SeO₃ | 0,01-0,5 mg/l |
| NiSO₄.6H₂O | 0,5-5 mg/l |
| CuSO₄.5H₂0 | 0,0025-1 mg/l |
| EDTA-Fe | 10-50 mg/l |
| Glukose | 20-60 g/l |
| (NH₄)₂SO₄ | 2-9 g/l |
| Thiamin | 1-50 mg/l |
| Vitamin B12 | 0,025-5 mg/l |
| Panthothenat | 0,1-25 mg/l |

14. Verwendung des Kulturmediums nach Anspruch 13 für die Kultivierung von Protisten zur Herstellung von Lipiden und Pigmenten.

15. Organismus der Gattung *Aurantiochytrium,* der dem Stamm FCC 1324 entspricht, der bei der CCAP (Culture Collection of Algae and Protozoa) unter der Zugangsnummer CCAP 4062/1 hinterlegt ist.

## Claims

1. A process for producing docosahexaenoic acid (DHA) comprising the following step:
the culture, under heterotrophic or mixotrophic conditions, of one or more strains of the genus *Aurantiochytrium* in a culture medium having less than 3.5 g/L of sodium ions,
**characterized in that** the strains of the genus *Aurantiochytrium* have a genetic identity of at least 92% to the SEQ NO. 1 sequence and
the culture medium is a chemically defined culture medium having less than 3.5 g/L of sodium ions and less than 1 g/L of chloride ions and having 200 mM to 500 mM of organic carbon substrate.

2. A culture process according to claim 1, **characterized in that** the strain(s) of the genus *Aurantiochytrium* also have a genetic identity of at least 96% to the SEQ NO. 2 sequence, and/or at least 91% to the SEQ NO. 3 sequence and/or at least 95% to the SEQ NO. 4 sequence.

3. A culture process according to claim 1 or 2, **characterized in that** the culture medium has less than 1 g/L, preferably less than 100 mg/L, more preferably less than 6 mg/L of sodium ions.

4. A process according to any one of claims 1 to 3, **characterized in that** the culture medium has less than 500 mg/L, preferably less than 200 mg/L of chloride ions.

5. A process according to any one of claim 1 to 4, **characterized in that** the medium does not comprise an osmotic pressure regulating agent, such as mannitol, sorbitol, polyethylene glycol and sucrose.

6. A process according to any one of claims 1 to 5, further comprising the steps:
b) of maintaining said culture over several generations,
c) recovery of the biomass thus cultivated,
d) recovery of lipids from the strains, and possibly,
e) the extraction of DHA (docosahexaenoic acid).

7. A process according to any one of the claims 1 to 6, **characterized in that** the culture medium consists of:
| Ingredients | Concentration |
|---|---|
| KCl | 0,05-0,5 g/l |
| H₃BO₃ | 0,01-0,3 g/l |
| MgSO₄, 7H₂0 | 2-10 g/l |
| CaCl₂, 2H₂0 | 0,2-0,9 g/l |
| KNO₃ | 0,01-0,06 g/l |
| KH₂PO₄, 7H2O | 0,2-1 g/l |
| Na₂EDTA, 2H₂O | 0,001-0,005 g/l |
| ZnSO₄.7H₂O | 0,01-0,1 mg/l |
| CoCl₂.6H₂O | 0,01-0,1 mg/l |
| MnCl₂.4H₂O | 0,05-1 mg/l |
| Na₂MoO₄, 2H₂O | 0,0005-0,1 mg/l |
| Na₂SeO₃ | 0,01-0,5 mg/l |
| NiSO₄.6H₂O | 0,5-5 mg/l |
| CuSO₄.5H₂0 | 0,0025-1 mg/l |
| EDTA-Fe | 10-50 mg/l |
| Glucose | 20-60 g/l |
| (NH₄)₂SO₄ | 2-9 g/l |
| Thiamine | 1-50 mg/l |
| Vitamin B12 | 0,025-5 mg/l |
| Panthothenate | 0,1-25 mg/l |

8. A process according to claim 7, **characterized in that** the biomass from step b) represents at least 100 g/L of dry matter.

9. A process according to claim 7 or 8, **characterized in that** the concentration of DHA at the end of step b) represents at least 15 g/L.

10. A process according to any one of claims 7 to 9, **characterized in that** the DHA contained in the biomass at the end of step b) represents more than 30% of the total lipids.

11. A process according to any one of claims 1 to 10, **characterized in that** said organism of the genus *Aurantiochytrium* corresponds to strain FCC 1324, deposited with the CCAP (Culture Collection of Algae and Protozoa), under accession number CCAP 4062/1.

12. Use of all or part of the biomass obtained from the process according to any one of claims 1 to 11 as a product, ingredient in a product for human consumption or as raw material for animal feed, in particular aquaculture.

13. A culture medium **characterized in that** it consists of:
| Ingredients | Concentration |
|---|---|
| KCl | 0,05-0,5 g/l |
| H₃BO₃ | 0,01-0,3 g/l |
| MgSO₄, 7H₂0 | 2-10 g/l |
| CaCl₂, 2H₂0 | 0,2-0,9 g/l |
| KNO₃ | 0,01-0,06 g/l |
| KH₂PO₄, 7H2O | 0,2-1 g/l |
| Na₂EDTA, 2H₂O | 0,001-0,005 g/l |
| ZnSO₄.7H₂O | 0,01-0,1 mg/l |
| CoCl₂.6H₂O | 0,01-0,1 mg/l |
| MnCl₂.4H₂O | 0,05-1 mg/l |
| Na₂MoO₄, 2H₂O | 0,0005-0,1 mg/l |
| Na₂SeO₃ | 0,01-0,5 mg/l |
| NiSO₄.6H₂O | 0,5-5 mg/l |
| CuSO₄.5H₂0 | 0,0025-1 mg/l |
| EDTA-Fe | 10-50 mg/l |
| Glucose | 20-60 g/l |
| (NH₄)₂SO₄ | 2-9 g/l |
| Thiamine | 1-50 mg/l |
| Vitamin B12 | 0,025-5 mg/l |
| Panthothenate | 0,1-25 mg/l |

14. Use of the culture medium according to claim 13 for the culture of protists for the production of lipids and pigments.

15. Organism of the genus *Aurantiochytrium* corresponding to the strain FCC 1324, deposited with the CCAP (Culture Collection of Algae and Protozoa), under accession number CCAP 4062/1.
